(19) **Europäisches Patentamt / European Patent Office / Office européen des brevets**

(11) **EP 4 585 144 B1**

(12) **EUROPEAN PATENT SPECIFICATION**

(45) Date of publication and mention
of the grant of the patent:
**22.04.2026  Bulletin 2026/17**

(21) Application number: **25177545.8**

(22) Date of filing: **09.05.2023**

(51) International Patent Classification (IPC):
**A61B 5/02** *(2006.01)*  **A61B 5/029** *(2006.01)*
**A61B 5/11** *(2006.01)*  **A61B 5/00** *(2006.01)*

(52) Cooperative Patent Classification (CPC):
**A61B 5/02028; A61B 5/029; A61B 5/1107;**
**A61B 5/6822; A61B 5/7275; A61B 5/7282;**
A61B 2505/01; A61B 2562/0219

(54) **A DETECTOR SYSTEM AND A METHOD FOR DETECTING EXACERBATION OF HEART HYPOFUNCTION**

DETEKTORSYSTEM UND VERFAHREN ZUR ERKENNUNG EINER VERSCHLIMMERUNG DER HERZHYPOFUNKTION

SYSTÈME DE DÉTECTEUR ET PROCÉDÉ DE DÉTECTION D'EXACERBATION D'HYPOFONCTION CARDIAQUE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Validation States:
**TN**

(30) Priority: **20.05.2022  FI 20225450**

(43) Date of publication of application:
**16.07.2025  Bulletin 2025/29**

(62) Document number(s) of the earlier application(s) in accordance with Art. 76 EPC:
**23724005.6 / 4 525 703**

(73) Proprietor: **Precordior Oy**
**20100 Turku (FI)**

(72) Inventors:
• **KOIVISTO, Tero**
  **20014 Turun yliopisto (FI)**
• **KAISTI, Matti**
  **20014 Turun yliopisto (FI)**
• **PÄNKÄÄLÄ, Mikko**
  **20014 Turun yliopisto (FI)**
• **SIRKIÄ, Jukka-Pekka**
  **20014 Turun yliopisto (FI)**
• **PANULA, Tuukka**
  **20014 Turun yliopisto (FI)**

(74) Representative: **Väänänen, Janne Kalervo**
**Vanarix Oy**
**Laaksolahdentie 74**
**02730 Espoo (FI)**

(56) References cited:
US-A1- 2015 106 020    US-A1- 2017 296 119
US-A1- 2021 259 560    US-A1- 2021 407 276
US-A1- 2022 071 561

## Description

### Field of the disclosure

The disclosure relates to a detector system and to a method for detecting exacerbation of heart hypofunction. Furthermore, the disclosure relates to computer program for detecting exacerbation of heart hypofunction.

### Background

Abnormalities that may occur in a cardiovascular system, if not diagnosed and appropriately treated and/or remedied, may progressively decrease the health of an individual. For example, pulmonary hypertension "PAH" represents in many cases an early indication for an oncoming exacerbation phase of heart hypofunction that will take place in average after from three to four weeks from the occurrence of the pulmonary hypertension. In many cases, the pulmonary hypertension can predict the exacerbation phase of heart hypofunction in a so early stage that traditional indications of heart hypofunction such as e.g. increase in weight and increase in blood pressure are typically not present. Heart hypofunction diagnosed at an early phase can often be treated and/or remedied and thereby mortality and a need for hospitalization can decrease significantly.

Known devices for determining the state of a subject are disclosed by US 2021/0259560 A1, US 2018/0106020 A1, US 2017/0296119 A1, US 2022/0071561 A1, and US 2021/0407276 A1.

Abbott Laboratories, Chicago USA, has developed a system for detecting exacerbation of heart hypofunction. The system comprises a micromechanical sensor and a receiver that receives measurement data from the micromechanical sensor. The micromechanical sensor is placed inside the pulmonary artery via the right side of a heart. Thus, the method of Abbott Laboratories for detecting exacerbation of heart hypofunction is an invasive method. The receiver of the system of Abbott Laboratories can be placed e.g. on a bed of a patient. When the patient is lying on the bed, the micromechanical sensor sends measurement data via the receiver to a cloud service. The measurement data can be indicative of the above-mentioned pulmonary hypertension "PAH" that represents in many cases an early indication for an oncoming exacerbation phase of heart hypofunction.

Invasive methods of the kind described above have their natural risks related to a need for invasive operations on a human body. Furthermore, some invasive methods can be used during surgical operations only. On the other hand, a non-invasive method based on Doppler-ultrasound measurement requires expensive equipment and a skilful and experienced examining personnel. Thus, there is a need for non-invasive systems and methods for detecting exacerbation of heart hypofunction.

### Summary

In this document, the word "geometric" when used as a prefix means a geometric concept that is not necessarily a part of any physical object. The geometric concept can be for example a geometric point, a straight or curved geometric line, a geometric plane, a non-planar geometric surface, a geometric space, or any other geometric entity that is zero, one, two, or three dimensional.

In accordance with the invention, there is provided a new detector system for non-invasively detecting exacerbation of heart hypofunction. A detector system according to the invention comprises:

- a processing system configured to receive a measurement signal,
- a sensor, e.g. a gyroscope, configured to produce the measurement signal when being in a movement sensing relation with a jugular vein "JV", lat. vena jugularis, of an individual, and
- a memory system communicatively connected to the processing system.

The processing system is configured to:

- compare first data stored in the memory system and based on an earlier produced first part of the measurement signal to second data based on a later produced second part of the measurement signal,

- compute a frequency spectrum of the measurement signal,

- compute energy of a part of the frequency spectrum above a predetermined frequency limit, and

- set an indicator data to express the exacerbation of heart hypofunction in response to a situation in which the comparison between the first data and the second data expresses an increase of the energy of the part of the frequency spectrum above the predetermined frequency limit.

The jugular vein is directly connected to the right atrium of a heart, and thus variation in the jugular vein pressure is produced by changes in blood flow and changes in pressure caused by fillings and contractions of the right atrium and the right ventricle of the heart. This opens a door for a non-invasive examination directed to the right side of a heart, i.e. the right ventricle and the right atrium, based on changes in the behaviour of the jugular vein pressure. In the system according to the invention, possible changes in the behaviour of the jugular vein pressure are detected by comparing the first data stored in the memory system and based on the earlier produced part of the measurement signal to the second data based on the later produced part of the measurement signal. In addition to the above-mentioned first and second parts of the measurement signal, it is possible to produce third, fourth, etc. parts of the measurement signal and thereby to monitor the development of the heart hypofunction situation over time.

The above-mentioned sensor is advantageously a rotation sensor which can be against the skin of an individual and in a movement sensing relation with a jugular vein of the individual. The rotation sensor is advantageously positioned so that one end of the rotation sensor is nearer to the jugular vein than another end of the rotation sensor. Thus, variation in the jugular vein pressure causes more movement at the first-mentioned end of the rotation sensor than at the last-mentioned end of the rotation sensor, and this difference appears as rotational movement of the rotation sensor. A movement which is not related to the jugular vein pressure and which has a substantially same amplitude and direction over a whole skin area covered by the rotation sensor does not cause a significant rotational movement of the rotation sensor but a translational movement only, and thereby this movement does not cause a significant signal component in the output signal of the rotation sensor. Therefore, the rotation sensor that measures rotation is more insensitive to many movements not related to the variation of the jugular vein pressure than for example an acceleration sensor that measures translational movements.

In accordance with the invention, there is also provided a new method that comprises:

- maintaining a memory system storing a measurement signal that has been produced with a sensor that is in a movement sensing relation with a jugular vein of an individual,

- comparing first data stored in the memory system and based on an earlier produced first part of the measurement signal to second data based on a later produced second part of the measurement signal,

- computing a frequency spectrum of the measurement signal,

- computing energy of a part of the frequency spectrum above a predetermined frequency limit, and

- setting an indicator data to express exacerbation of heart hypofunction in response to a situation in which the comparison between the first data and the second data expresses an increase of the energy of the part of the frequency spectrum above the predetermined frequency limit.

In accordance with the invention, there is also provided a new computer program for controlling a programmable data processing system to detect exacerbation of heart hypofunction. The computer program comprises computer executable instructions for controlling the programmable data processing system to:

- receive a measurement signal from a sensor suitable for producing the measurement signal when being in a movement sensing relation with a jugular vein of an individual,

- store, to a memory system, first data that is based on an earlier produced first part of the measurement signal,

- compare the first data to second data that is based on a later produced second part of the measurement signal,

- compute a frequency spectrum of the measurement signal,

- compute energy of a part of the frequency spectrum above a predetermined frequency limit, and

- set an indicator data to express the exacerbation of heart hypofunction in response to a situation in which the comparison between the first data and the second data expresses an increase of the energy of the part of the frequency spectrum above the predetermined frequency limit.

In accordance with the invention, there is provided also a new computer program product. The computer program product comprises a non-volatile computer readable medium, e.g. a compact disc "CD", encoded with a computer program according to the invention.

Exemplifying and non-limiting embodiments are described in accompanied dependent claims.

Various exemplifying and non-limiting embodiments both as to constructions and to methods of operation, together with additional objects and advantages thereof, will be best understood from the following description of specific exemplifying embodiments when read in conjunction with the accompanying drawings.

The verbs "to comprise" and "to include" are used in this document as open limitations that neither exclude nor require the existence of also un-recited features.

The features recited in the accompanied dependent claims are mutually freely combinable unless otherwise explicitly stated.

Furthermore, it is to be understood that the use of "a" or "an", i.e. a singular form, throughout this document does not exclude a plurality.

**Brief description of figures**

Exemplifying and non-limiting embodiments and their advantages are explained in greater detail below with reference to the accompanying drawings, in which:

figure 1 illustrates a detector system according to an exemplifying and non-limiting embodiment for non-invasively detecting exacerbation of heart hypofunction,

figures 2a and 2b illustrate functionality of a detector system according to an exemplifying and non-limiting embodiment for non-invasively detecting exacerbation of heart hypofunction,

figure 3 shows exemplifying waveforms of jugular vein pressure "JVP" and an electrocardiogram "ECG" to illustrate functionality of a detector system according to an exemplifying and non-limiting embodiment for non-invasively detecting exacerbation of heart hypofunction, and

figure 4 shows a flowchart of a method according to an exemplifying and non-limiting embodiment for non-invasively detecting exacerbation of heart hypofunction.

**Description of exemplifying and non-limiting embodiments**

The specific examples provided in the description below should not be construed as limiting the scope and/or the applicability of the appended claims. Lists and groups of examples provided in the description are not exhaustive unless otherwise explicitly stated.

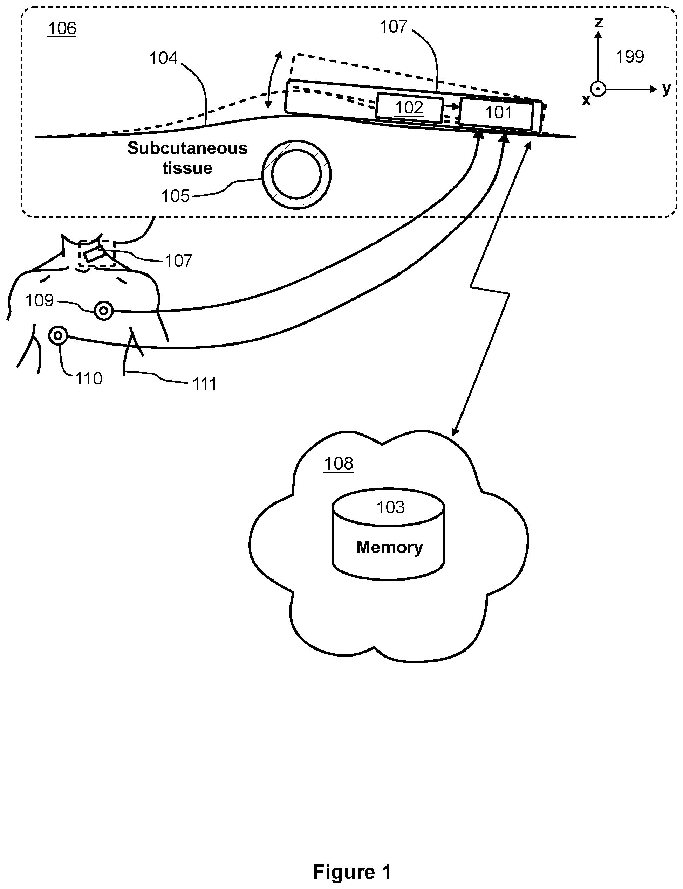

Figure 1 illustrates a detector system according to an exemplifying and non-limiting embodiment for non-invasively detecting exacerbation of heart hypofunction. The detector system comprises a sensor 102 which is configured to produce a measurement signal when the sensor 102 is in a movement sensing relation with a jugular vein 105 of an individual 111. In the exemplifying detector system illustrated in figure 1, the sensor 102 is a rotation sensor, e.g. a gyroscope, configured to produce the measurement signal indicative of rotation of the rotation sensor when the rotation sensor is against a skin 104 of the individual 111 so that the rotation sensor is in the movement sensing relation with the jugular vein 105 of the individual. It is however also possible that the sensor is for example an optical sensor suitable for optically measuring movement caused by the jugular vein 105 on the skin 104. In a part 106 of figure 1, the direction perpendicular to the skin 104 is substantially parallel with the z-axis of a coordinate system 199. In this exemplifying case, the sensor 102 is a part of a device 107 that is placed against the skin 104 of the individual 111. The device 107 can be for example a mobile phone. The detector system comprises a processing system 101 configured to receive the measurement signal from the sensor 102. Furthermore, the detector system comprises a memory system 103 that is communicatively connected to the processing system 101. In this exemplifying case, the memory system 103 is implemented as a cloud service in an external data network 108.

The jugular vein 105 is directly connected to the right atrium of a heart, and thus variation in the jugular vein pressure "JVP" is produced by changes in blood flow and changes in pressure caused by fillings and contractions of the right atrium and the right ventricle of the heart. This opens a door for a non-invasive examination directed to the right side of a heart, i.e. the right ventricle and the right atrium, based on changes in the behaviour of the jugular vein pressure. To detect possible changes in the behaviour of the jugular vein pressure, the processing system 101 is configured to compare first data stored in the memory system 103 and based on an earlier produced first part of the measurement signal to second data based on a later produced second part of the measurement signal. Furthermore, the processing system 101 is configured to form indicator data indicative of the exacerbation of heart hypofunction based on the comparison between the first data and the second data. The device 107 may comprise for example a display for presenting the indicator data to a user. The display is not shown in figure 1. It is also possible that the device is configured to send the indicator data to the data network 108. The above-mentioned first and second data can be defined in different ways based on the first and second parts of the measurement signal. Some examples are presented below, but it is worth noting that the invention is not limited to the belowpresented examples.

In a detection system according to an exemplifying and non-limiting embodiment, the sensor 102 is a rotation sensor configured to measure angular velocity ω of the rotation sensor. In this exemplifying case, the first data can be a peak value $\omega_{max1}$ of the angular velocity in a first measurement, and the second data can be a peak value $\omega_{max2}$ of the angular velocity in a second measurement made after the first measurement. The processing system 101 is configured to set the indicator data to express the exacerbation of heart hypofunction in response to a situation in which the peak value $\omega_{max2}$ exceeds the peak value $\omega_{max1}$ with a predetermined margin.

In a detection system according to an exemplifying and non-limiting embodiment, the sensor 102 is a rotation sensor configured to measure angular acceleration α of the rotation sensor, and the processing system 101 is configured to compute a time integral of the angular α acceleration to estimate the angular velocity ω as a function of time t:

$$\omega(t) \;=\; \int_{t0}^{t} \alpha(u)du, \tag{1}$$

where t0 is the beginning of a measurement period under consideration. In this exemplifying case, the first data can be a peak value $\omega_{max1}$ of the angular velocity in a first measurement, and the second data can be a peak value $\omega_{max2}$ of the angular velocity in a second measurement made after the first measurement. The processing system 101 is configured to set the indicator data to express the exacerbation of heart hypofunction in response to a situation in which the peak value $\omega_{max2}$ exceeds the peak value $\omega_{max1}$ with a predetermined margin.

In a detection system according to an exemplifying and non-limiting embodiment, the sensor 102 is a three-axis gyroscope, and the processing system 101 is configured to compute total angular velocity of the three-axis gyroscope according to the following formula:

$$\omega_{xyz}(t) = (\omega_x{}^2 + \omega_y{}^2 + \omega_z{}^2)^{½} \tag{2}$$

where $\omega_{xyz}(t)$ is the total angular velocity as a function of time t, $\omega_x$ is angular velocity measured by the three-axis gyroscope around a geometric axis parallel with the x-axis of the coordinate system 199, $\omega_y$ is angular velocity measured by the three-axis gyroscope around a geometric axis parallel with the y-axis of the coordinate system 199, and $\omega_z$ is angular velocity measured by the three-axis gyroscope around a geometric axis parallel with the z-axis of the coordinate system 199. In this exemplifying case, the first data can be a peak value $\omega_{xyzmax1}$ of the computed total angular velocity in a first measurement, and the second data can be a peak value $\omega_{xyzmax2}$ of the computed total angular velocity in a second measurement made after the first measurement. The processing system 101 is configured to set the indicator data to express the exacerbation of heart hypofunction in response to a situation in which the peak value $\omega_{xyzmax2}$ exceeds the peak value $\omega_{xyzmax1}$ with a predetermined margin.

In a detection system according to an exemplifying and non-limiting embodiment, the processing system is configured to compute angular displacement $\theta_{xyz}(t)$ of the three-axis gyroscope according to the following formula:

$$\theta_{xyz}(t) = \int_{t0}^{t} \omega_{xyz}(u)du \tag{3}$$

where t0 is the beginning of a measurement period under consideration. In this exemplifying case, the first data can be a peak-to-peak value $(\theta_{xyz}(t)_{max} - \theta_{xyz}(t)_{min})$ of the angular displacement in a first measurement, and the second data can be the corresponding peak-to-peak value in a second measurement made after the first measurement. The processing system 101 is configured to set the indicator data to express the exacerbation of heart hypofunction in response to a situation in which the peak-to-peak value of the second measurement exceeds the peak-to-peak value of the first measurement with a predetermined margin.

In a detector system according to an exemplifying and non-limiting embodiment, the processing system 101 is configured to receive electric signals from electrodes 109 and 110 on the skin of the individual 111 and the processing system 101 is configured to produce an electrocardiogram "ECG" for a time interval of each measurement carried out by the sensor 102. The ECG signal can be utilized to improve the determination of an exacerbation phase of heart hypofunction.

The processing system 101 can be implemented for example with one or more processor circuits, each of which can be a programmable processor circuit provided with appropriate software, a dedicated hardware processor such as for example an application specific integrated circuit "ASIC", or a configurable hardware processor such as for example a field programmable gate array "FPGA". It is also possible that the device 107 comprises a memory system so that the device 107 can operate autonomously without a connection to the data network 108. The memory system of the device 107 may comprise for example one or more memory circuits each of which can be e.g. a random-access memory "RAM" circuit.

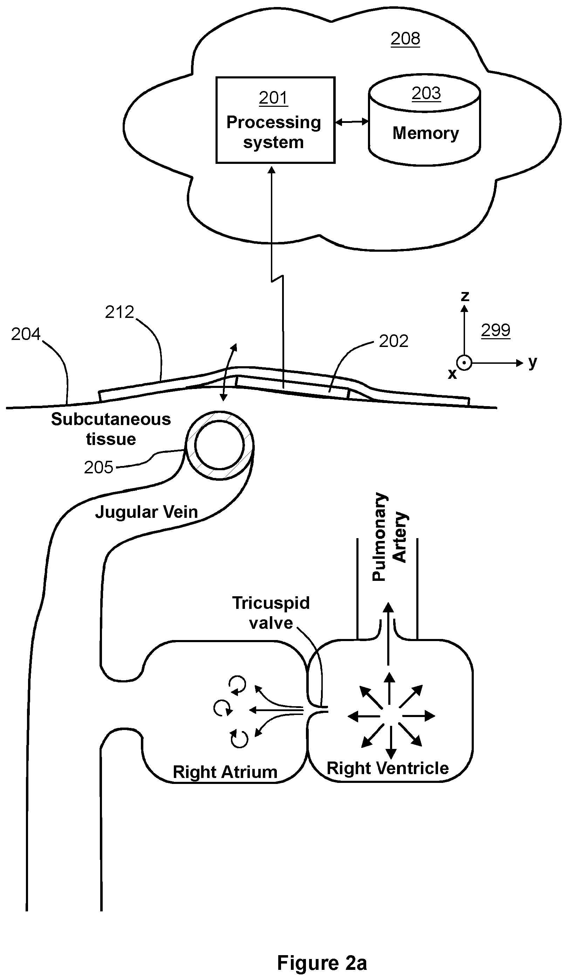

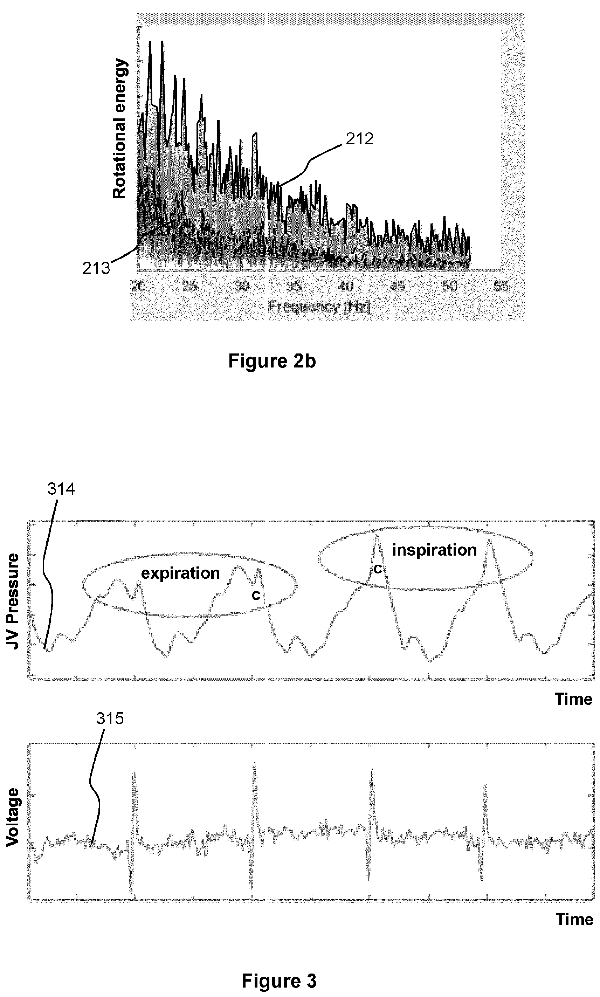

Figure 2a illustrates a detector system according to an exemplifying and non-limiting embodiment for non-invasively detecting exacerbation of heart hypofunction. Furthermore, figure 2a shows schematically the right side of a heart. The detector system comprises a sensor 202, e.g. a gyroscope, configured to produce a measurement signal when the sensor 202 is in a movement sensing relation with a jugular vein 205 of the individual. In figure 2, the direction perpendicular to the skin 204 is substantially parallel with the z-axis of a coordinate system 299. The detector system comprises a processing system 201 configured to receive the measurement signal from the sensor 202. The detector system comprises a memory system 203 communicatively connected to the processing system 201. In this exemplifying case, the processing system 201 and the memory system 203 are implemented as cloud services in an external data network 208.

The exemplifying detector system illustrated in figure 2 comprises a sheet of flexible material 212 that is provided with glue to attach the sensor 202 to the skin 204 of the individual. Thus, the sensor 202 can be used in different positions of the individual, e.g. when the individual is standing. The sensor 202 is configured to maintain a wireless link to transfer the measurement signal from the sensor 202 to a gateway, router, or some other suitable element of the data network 208. The wireless link can be for example a radio link such as e.g. a Bluetooth® link or a Near Field Communication "NFC" link. It is also possible that the wireless link is an optical or infrared link.

Figure 2a shows schematically the right side of a heart during a systolic phase. A leakage through the tricuspid valve during systolic phases is increasing when the pressure in the right ventricle is increasing due to exacerbation of heart hypofunction. The leakage causes a turbulent backflow to the right atrium. This turbulent backflow causes high frequency oscillations in the waveform of the jugular vein pressure "JVP". Plot 212 in figure 2b shows a spectrum of gyroscope rotational energy in a case of heart hypofunction, and plot 213 shows a spectrum of gyroscope rotational energy in a normal case. Therefore, exacerbation of heart hypofunction can be detected based on changes in high frequency oscillations in the jugular vein pressure "JVP".

In the detector system illustrated in figure 2a, the processing system 201 is configured to compute a frequency spectrum of the measurement signal and to compute energy of a part of the frequency spectrum above a predetermined frequency limit that can be e.g. 20 Hz. In this exemplifying case, the first data can be the computed energy corresponding to a first measurement, and the second data can be the computed energy corresponding to a second measurement made after the first measurement. The processing system 201 is configured to set the indicator data to express the exacerbation of heart hypofunction in response to a situation in which the comparison between the first data and the second data expresses an increase in the computed energy.

Figure 3 shows an exemplifying waveform 314 of jugular vein pressure "JVP" and an exemplifying waveform 315 of an electrocardiogram "ECG". The waveform 314 shows the jugular vein pressure during an exhale phase "expiration" and during an inhale phase "inspiration", too. During inhale phases, intra-thoracic pressure in the thoracic cavity decreases and thus more blood can enter the right atrium of a heart. As a corollary, the jugular vein is partially emptied. This, in turn, makes pulsations of the pulmonary artery to conduct better to a rotational sensor and/or another movement sensor. Especially, the c-wave of the jugular vein pulse is modified by the rapid increase of the pulmonary artery pressure during a systolic phase. In a case of heart hypofunction when the pressure in the right atrium is higher, the decrease in the intra-thoracic pressure does not empty the jugular vein in the same extend as in a normal case. Thus, in a case of heart hypofunction, the breathing cycle does not modulate the output signal of the rotational sensor and/or another movement sensor is the same way as in a normal case. Therefore, exacerbation of heart hypofunction can be detected based on changes in the modulation caused by the breathing cycle.

In a detector system according to an exemplifying and non-limiting embodiment, the processing system is configured to receive a signal indicative of inhale and exhale phases of breathing of an individual and to detect modulation of the measurement signal caused by the alternating inhale and exhale phases. In this exemplifying case, the first data can be the modulation detected during a first measurement, and the second data can be the modulation detected during a second measurement made after the first measurement. The modulation can be expressed e.g. as a difference between amplitudes, powers, etc. of the measurement signals during exhale and inhale phases. The processing system is configured to set the indicator data to express an exacerbation phase of heart hypofunction in response to a situation in which the comparison between the first data and the second data expresses a weakening of the modulation.

In a detector system according to an exemplifying and non-limiting embodiment, sensor fusion is utilized i.e. different sensors are used to produce the measurement signal dependent on the jugular vein pressure. For example, an acceleration sensor can be used together with a gyroscope so that drifting of a signal level typical to certain gyroscopes is corrected with the aid of an acceleration sensor and e.g. a Kalman filter.

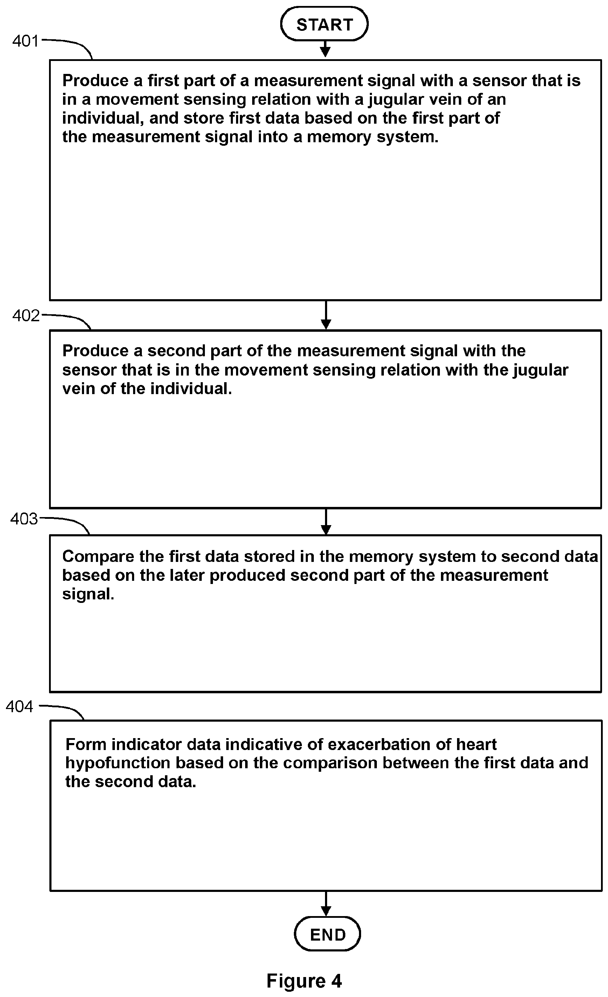

Figure 4 shows a flowchart of a method according to an exemplifying and non-limiting embodiment for non-invasively detecting exacerbation of heart hypofunction. The method comprises the following actions:

- action 401: producing a first part of a measurement signal with a sensor that is in a movement sensing relation with a jugular vein of an individual, and storing first data based on the first part of the measurement signal into a memory system,

- action 402: producing a second part of the measurement signal with the sensor that is in the movement sensing relation with the jugular vein of the individual,

- action 403: comparing the first data stored in the memory system and based on the earlier produced first part of the measurement signal to second data based on the later produced second part of the measurement signal, and

- action 404: forming indicator data indicative of exacerbation of heart hypofunction based on the comparison between the first data and the second data.

In a method according to an exemplifying and non-limiting embodiment, the sensor is a rotation sensor that produces the measurement signal indicative of rotation of the rotation sensor when being against a skin of the individual and in the movement sensing relation with the jugular vein of the individual.

In a method according to an exemplifying and non-limiting embodiment, the sensor is a rotation sensor that measures angular velocity of the rotation sensor, and the method comprises setting the indicator data to express the exacerbation of heart hypofunction in response to a situation in which the comparison between the first data and the second data expresses an increase of a peak value of the angular velocity.

In a method according to an exemplifying and non-limiting embodiment, the sensor is a rotation sensor that measures angular acceleration of the rotation sensor, and the method comprises computing a time integral of the measured angular acceleration and setting the indicator data to express the exacerbation of heart hypofunction in response to a situation in which the comparison between the first data and the second data expresses an increase of a peak value of the computed time integral.

In a method according to an exemplifying and non-limiting embodiment, the sensor comprises a gyroscope so that one or more output signals of the gyroscope represent the measurement signal. In a method according to an exemplifying and non-limiting embodiment, the gyroscope is a three-axis gyroscope, and the method comprises computing total angular velocity of the three-axis gyroscope according to the following formula:

$$\omega_{xyz}(t) = \left(\omega_x^2 + \omega_y^2 + \omega_z^2\right)^{\frac{1}{2}} \qquad (4)$$

where $\omega_{xyz}(t)$ is the total angular velocity as a function of time t, $\omega_x$ is angular velocity measured by the three-axis gyroscope in x-direction, $\omega_y$ is angular velocity measured by the three-axis gyroscope in y-direction, and $\omega_z$ is angular velocity measured by the three-axis gyroscope in z-direction. The indicator data is set to express the exacerbation of heart hypofunction in response to a situation in which the comparison between the first data and the second data expresses an increase of a peak value of the computed total angular velocity.

A method according to an exemplifying and non-limiting embodiment comprises computing angular displacement of the three-axis gyroscope according to the following formula:

$$\theta_{xyz}(t) = \int_{t0}^{t} \omega_{xyz}(u)du \qquad (5)$$

where $\theta_{xyz}(t)$ is the angular displacement as a function of time t and t0 is a starting point of a measurement period under consideration, and the method comprises setting the indicator data to express the exacerbation of heart hypofunction in response to a situation in which the comparison between the first data and the second data expresses an increase of a peak-to-peak value $(\theta_{xyz}(t)_{max} - \theta_{xyz}(t)_{min})$ of the angular displacement.

A method according to an exemplifying and non-limiting embodiment comprises computing a frequency spectrum of the measurement signal, computing energy of a part of the frequency spectrum above a predetermined frequency limit, and setting the indicator data to express the exacerbation of heart hypofunction in response to a situation in which the comparison between the first data and the second data expresses an increase in the energy of the part of the frequency spectrum.

A method according to an exemplifying and non-limiting embodiment comprises receiving a signal indicative of inhale and exhale phases of breathing of the individual, detecting modulation of the measurement signal caused by the alternating inhale and exhale phases, and setting the indicator data to express the exacerbation of heart hypofunction in response to a situation in which the comparison between the first data and the second data expresses a weakening of the modulation.

A method according to an exemplifying and non-limiting embodiment comprises receiving one or more electric signals from electrodes on the skin of the individual and producing electrocardiograms "ECG" for time intervals corresponding to the first and second data.

In method according to an exemplifying and non-limiting embodiment, the sensor maintains a wireless link to transfer the measurement signal from the sensor to a processing system configured to form the indicator data.

In method according to an exemplifying and non-limiting embodiment, the sensor is a part of a mobile phone.

A computer program according to an exemplifying and non-limiting embodiment comprises computer executable instructions for controlling a programmable data processing system to carry out actions related to a method according to any of the above-described exemplifying and non-limiting embodiments.

A computer program according to an exemplifying and non-limiting embodiment comprises software modules for controlling a programmable data processing system to detect exacerbation of heart hypofunction. The software modules comprise computer executable instructions for controlling the programmable data processing system to:

- receive a measurement signal from a sensor suitable for producing the measurement signal when being in a movement sensing relation with a jugular vein of an individual,
- store, to a memory system, first data based on a first part of the measurement signal,
- compare the first data to second data based on a later produced second part of the measurement signal, and
- form indicator data indicative of the exacerbation of heart hypofunction based on the comparison between the first data and the second data.

The software modules can be for example subroutines or functions implemented with programming tools suitable for the programmable data processing system.

A computer program product according to an exemplifying and non-limiting embodiment comprises a computer readable medium, e.g. a compact disc "CD", encoded with a computer program according to an exemplifying embodiment of the invention.

A signal according to an exemplifying and non-limiting embodiment is encoded to carry information defining a computer program according to an exemplifying embodiment. The specific examples provided in the description given above should not be construed as limiting the scope and/or the applicability of the appended claims. Lists and groups of examples provided in the description given above are not exhaustive unless otherwise explicitly stated. Correspondingly, exemplifying waveforms and other exemplifying results presented above and/or in figures should not be construed as limiting the scope and/or the applicability of the appended claims.

**Claims**

1. A detector system for detecting exacerbation of heart hypofunction, the detector system comprising:

> - a processing system (101, 201) configured to receive a measurement signal,
> - a sensor (102, 202) configured to produce the measurement signal when being in a movement sensing relation with a jugular vein of an individual, and
> - a memory system (103, 203) communicatively connected to the processing system,
> wherein the processing system is configured to:
>
>> - compare first data stored in the memory system and based on an earlier produced first part of the measurement signal to second data based on a later produced second part of the measurement signal, and
>> - form indicator data indicative of the exacerbation of heart hypofunction based on the comparison between the first data and the second data,
>
> **characterized in that** the processing system is configured to compute a frequency spectrum of the measurement signal, to compute energy of a part of the frequency spectrum above a predetermined frequency limit, and to set the indicator data to express the exacerbation of heart hypofunction in response to a situation in which the comparison between the first data and the second data expresses an increase of the energy of the part of the frequency spectrum.

2. A detector system according to claim 1, wherein the processing system is configured to receive a signal indicative of inhale and exhale phases of breathing of the individual, to detect modulation of the measurement signal caused by the alternating inhale and exhale phases, and to set the indicator data to express the exacerbation of heart hypofunction in response to a situation in which the comparison between the first data and the second data expresses a weakening of the modulation.

3. A detector system according to claim 1 or 2, wherein the processing system is configured to receive one or more electric signals from electrodes on the skin of the individual and the processing system is configured to produce electrocardiograms for time intervals corresponding to the first and second parts of the measurement signal.

4. A detector system according to any one of claims 1-3, wherein the processing system and the sensor are configured to maintain a wireless link to transfer the measurement signal from the sensor to the processing system.

5. A detector system according to any one of claims 1-4, wherein the sensor (102) is a part of a mobile phone.

6. A method comprising:

- maintaining a memory system storing a measurement signal that has been produced with a sensor that is in a movement sensing relation with a jugular vein of an individual,
- comparing (403) first data stored in the memory system and based on an earlier produced first part of the measurement signal to second data based on a later produced second part of the measurement signal, and
- forming (404) indicator data indicative of exacerbation of heart hypofunction based on the comparison between the first data and the second data,

**characterized in that** the method comprises computing a frequency spectrum of the measurement signal, computing energy of a part of the frequency spectrum above a predetermined frequency limit, and setting the indicator data to express the exacerbation of heart hypofunction in response to a situation in which the comparison between the first data and the second data expresses an increase of the energy of the part of the frequency spectrum.

7. A computer program for controlling a programmable data processing system to detect exacerbation of heart hypofunction, the computer program comprises computer executable instructions for controlling the programmable data processing system to:

- receive a measurement signal from a sensor suitable for producing the measurement signal when being in a movement sensing relation with a jugular vein of an individual,
- store, to a memory system, first data based on an earlier produced first part of the measurement signal,
- compare the first data to second data based on a later produced second part of the measurement signal, and
- form indicator data indicative of the exacerbation of heart hypofunction based on the comparison between the first data and the second data,

**characterized in that** the computer program further comprises computer executable instructions for controlling the programmable data processing system to compute a frequency spectrum of the measurement signal, to compute energy of a part of the frequency spectrum above a predetermined frequency limit, and to set the indicator data to express the exacerbation of heart hypofunction in response to a situation in which the comparison between the first data and the second data expresses an increase of the energy of the part of the frequency spectrum.

8. A non-transitory computer readable medium encoded with a computer program according to claim 7.

**Patentansprüche**

1. Ein Detektionssystem zur Erkennung einer Verschlechterung der Herzinsuffizienz, wobei das Detektionssystem folgendes umfasst:

- ein Verarbeitungssystem (101, 201), das zum Empfang eines Messignals konfiguriert ist,
- einen Sensor (102, 202), der so konfiguriert ist, dass er das Messsignal erzeugt, wenn er sich in einer Bewegungserfassungsbeziehung mit einer Halsvene einer Person befindet, und
- ein Speichersystem (103, 203), das mit dem Verarbeitungssystem in Verbindung steht,
wobei das Verarbeitungssystem so konfiguriert ist, dass es:

- erste Daten, die im Speichersystem gespeichert sind und auf einem früher erzeugten ersten Teil des Messsignals basieren, mit zweiten Daten vergleicht, die auf einem später erzeugten zweiten Teil des Messsignals basieren, und
- Anzeigedaten bildet, die, auf der Grundlage des Vergleichs zwischen den ersten Daten und der zweiten Daten, die Verschlimmerung der Herzinsuffizienz anzeigen,

**dadurch gekennzeichnet, dass** das Verarbeitungssystem so konfiguriert ist, dass es ein Frequenzspektrum des Messsignals berechnet, die Energie eines Teils des Frequenzspektrums oberhalb einer vorbestimmten Frequenzgrenze berechnet, und die Indikator-Daten so einstellt, dass sie die Verschlimmerung der Herzinsuffizienz als Reaktion auf eine Situation ausdrücken, bei welcher der Vergleich zwischen den ersten Daten und den zweiten Daten einen Anstieg der Energie des Teils des Frequenzspektrums ausdrückt.

2. Detektionssystem nach Anspruch 1, wobei das Verarbeitungssystem so konfiguriert ist, dass es ein Signal empfängt, das die Ein- und Ausatmungsphasen der Atmung der Person anzeigt, die Modulation des Messsignals erkennt, die durch die abwechselnden Einund Ausatmungsphasen verursacht werden, und die Anzeigedaten so einstellt, dass sie die Verschlimmerung der Herzinsuffizienz als Reaktion auf eine Situation ausdrücken, in der der Vergleich zwischen den ersten Daten und den zweiten Daten eine Abschwächung der Modulation zum Ausdruck bringt.

3. Detektionssystem nach Anspruch 1 oder 2, wobei das Verarbeitungssystem so konfiguriert ist, dass es ein oder mehrere elektrische Signale von Elektroden auf der Haut der Person empfängt, und das Verarbeitungssystem so konfiguriert ist, dass es Elektrokardiogramme für Zeitintervalle erzeugt, die dem ersten und zweiten Teil des Messsignals entsprechen.

4. Detektionssystem nach einem der Ansprüche 1 bis 3, wobei das Verarbeitungssystem und der Sensor so konfiguriert sind, dass sie eine drahtlose Verbindung aufrechterhalten, um das Messsignal vom Sensor zum Verarbeitungssystem zu übertragen.

5. Detektionssystem nach einem der Ansprüche 1 bis 4, wobei der Sensor (102) Teil eines Mobiltelefons ist.

6. Verfahren, das Folgendes umfasst:

- Aufrechterhalten eines Speichersystems, das ein Messsignal speichert, das mit einem Sensor erzeugt wurde, der sich in einer Bewegungserfassungsbeziehung zu einer Halsvene einer Person befindet,
- Vergleichen (403) erster Daten, die in dem Speichersystem gespeichert sind und auf einem früher erzeugten ersten Teil des Messsignals basieren, mit zweiten Daten, die auf einem später erzeugten zweiten Teil des Messsignals basieren, und
- Bilden (404) von Indikator-Daten, die eine Verschlimmerung der Herzinsuffizienz anzeigen, basierend auf dem Vergleich zwischen den ersten Daten und den zweiten Daten,

**dadurch gekennzeichnet, dass** das Verfahren das Berechnen eines Frequenzspektrums des Messsignals, das Berechnen der Energie eines Teils des Frequenzspektrums oberhalb einer vorbestimmten Frequenzgrenze, und das Einstellen der Indikator-Daten umfasst, um die Verschlimmerung der Herzinsuffizienz in Reaktion auf eine Situation auszudrücken, in welcher der Vergleich zwischen den ersten Daten und den zweiten Daten einen Anstieg der Energie des Teils des Frequenzspektrums ausdrückt.

7. Computerprogramm zur Steuerung eines programmierbaren Datenverarbeitungssystems zur Erkennung einer Verschlimmerung der Herzinsuffizienz, wobei das Computerprogramm computerausführbare Anweisungen zur Steuerung des programmierbaren Datenverarbeitungssystems umfasst, zum:

- Empfangen eines Messsignals von einem Sensor, der geeignet ist, das Messsignal zu erzeugen, wenn er sich in einer bewegungserfassenden Beziehung zu einer Halsvene einer Person befindet,
- Speichern erster Daten, die auf einem früher erzeugten ersten Teil des Messsignals basieren, in einem Speichersystem,
- Vergleichen der ersten Daten mit zweiten Daten, die auf einem später erzeugten zweiten Teil des Messsignals basieren, und
- Bilden von Indikator-Daten, die die Verschlimmerung der Herzinsuffizienz anzeigen, basierend auf dem Vergleich zwischen den ersten Daten und den zweiten Daten,

**EP 4 585 144 B1**

**dadurch gekennzeichnet, dass** das Computerprogramm ferner computerausführbare Anweisungen zum Steuern des programmierbaren Datenverarbeitungssystems umfasst, um ein Frequenzspektrum des Messsignals zu berechnen, um die Energie eines Teils des Frequenzspektrums oberhalb einer vorbestimmten Frequenzgrenze zu berechnen, und um die Indikator-Daten zur Darstellung der Verschlimmerung der Herzinsuffizienz in Reaktion auf eine Situation einzustellen, in welcher der Vergleich zwischen den ersten Daten und den zweiten Daten eine Zunahme der Energie des Teils des Frequenzspektrums zum Ausdruck bringt.

8. Nicht-transitorisches, computerlesbares Medium, das mit einem Computerprogramm nach Anspruch 7 codiert ist.

**Revendications**

1. Système de détecteur pour détecter l'exacerbation de l'hypofonction cardiaque, le système de détecteur comprenant :

   - un système de traitement (101, 201) configuré pour recevoir un signal de mesure,
   - un capteur (102, 202) configuré pour produire le signal de mesure quand il est dans une relation de capture de mouvement avec une veine jugulaire d'un individu et
   - un système de mémoire (103, 203) connecté de manière communicative au système de traitement,
   le système de traitement étant configuré pour :

      - comparer les premières données stockées dans le système de mémoire et basées sur une première partie produite plus tôt du signal de mesure aux secondes données basées sur une seconde partie produite plus tard du signal de mesure et
      - former des données d'indicateur indicatrices de l'exacerbation de l'hypofonction cardiaque sur la base de la comparaison entre les premières données et les secondes données,

      **caractérisé en ce que** le système de traitement est configuré pour calculer un spectre de fréquence du signal de mesure, pour calculer l'énergie d'une partie du spectre de fréquence au-dessus d'une limite de fréquence prédéterminée et pour régler les données d'indicateur pour exprimer l'exacerbation de l'hypofonction cardiaque en réponse à une situation dans laquelle la comparaison entre les premières données et les secondes données exprime une augmentation de l'énergie de la partie du spectre de fréquence.

2. Système de détecteur selon la revendication 1, dans lequel le système de traitement est configuré pour recevoir un signal indicateur des phases d'inhalation et d'exhalaison de la respiration de l'individu, pour détecter la modulation du signal de mesure provoquée par les phases d'inhalation et d'exhalaison en alternance et pour régler les données d'indicateur pour exprimer l'exacerbation de l'hypofonction cardiaque en réponse à une situation dans laquelle la comparaison entre les premières données et les secondes données exprime un affaiblissement de la modulation.

3. Système de détecteur selon la revendication 1 ou 2, dans lequel le système de traitement est configuré pour recevoir un ou plusieurs signaux électriques d'électrodes sur la peau de l'individu et le système de traitement est configuré pour produire des électrocardiogrammes durant des intervalles de temps correspondant aux première et seconde parties du signal de mesure.

4. Système de détecteur selon l'une quelconque des revendications 1 à 3, dans lequel le système de traitement et le capteur sont configurés pour maintenir un lien sans fil pour transférer le signal de mesure du capteur au système de traitement.

5. Système de détecteur selon l'une quelconque des revendications 1 à 4, dans lequel le capteur (102) est une partie d'un téléphone mobile.

6. Procédé comprenant :

   - le maintien d'un système de mémoire stockant un signal de mesure qui a été produit avec un capteur qui est dans une relation de capture de mouvement avec une veine jugulaire d'un individu,
   - la comparaison (403) des premières données stockées dans la mémoire et basées sur une première partie produite plus tôt du signal de mesure aux secondes données basées sur une seconde partie produite plus tard du signal de mesure et
   - la formation (404) des données d'indicateur indicatrices de l'exacerbation de l'hypofonction cardiaque sur la base de la comparaison entre les premières données et les secondes données,

**caractérisé en ce que** le procédé comprend le calcul d'un spectre de fréquence du signal de mesure, en calculant l'énergie d'une partie du spectre de fréquence au-dessus d'une limite de fréquence prédéterminée et en réglant les données d'indicateur pour exprimer l'exacerbation de l'hypofonction cardiaque en réponse à une situation dans laquelle la comparaison entre les premières données et les secondes données exprime une augmentation de l'énergie de la partie du spectre de fréquence.

7. Programme informatique pour commander un système de traitement de données programmables pour détecter l'exacerbation de l'hypofonction cardiaque, le programme informatique comprend des instructions exécutables sur ordinateur pour commander le système de traitement de données programmables à :

- recevoir un signal de mesure d'un capteur approprié pour produire le signal de mesure quand il est dans une relation de capture de mouvement avec une veine jugulaire d'un individu,
- stocker, dans un système de mémoire, les premières données basées sur une première partie produite plus tôt du signal de mesure,
- comparer les premières données aux secondes données basées sur une seconde partie produite plus tard du signal de mesure et
- former les données d'indicateur indicatrices de l'exacerbation de l'hypofonction cardiaque sur la base de la comparaison des premières données et des secondes données,

**caractérisé en ce que** le programme informatique comprend en outre des instructions exécutables sur ordinateur pour commander le système de traitement de données programmables pour calculer un spectre de fréquence du signal de mesure, pour calculer l'énergie d'une partie du spectre de fréquence au-dessus d'une limite de fréquence prédéterminée et pour régler les données d'indicateur pour exprimer l'exacerbation de l'hypofonction cardiaque en réponse à une situation dans laquelle la comparaison entre les premières données et les secondes données exprime une augmentation de l'énergie de la partie du spectre de fréquence.

8. Support lisible sur ordinateur non transitoire codé avec un programme informatique selon la revendication 7.

**Figure 1**

**Figure 2a**

**Figure 2b**

**Figure 3**

START

**401** — Produce a first part of a measurement signal with a sensor that is in a movement sensing relation with a jugular vein of an individual, and store first data based on the first part of the measurement signal into a memory system.

**402** — Produce a second part of the measurement signal with the sensor that is in the movement sensing relation with the jugular vein of the individual.

**403** — Compare the first data stored in the memory system to second data based on the later produced second part of the measurement signal.

**404** — Form indicator data indicative of exacerbation of heart hypofunction based on the comparison between the first data and the second data.

END

**Figure 4**

**EP 4 585 144 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20210259560 A1
- US 20180106020 A1
- US 20170296119 A1
- US 20220071561 A1
- US 20210407276 A1